# EUROPEAN PATENT SPECIFICATION

(11) **EP 1 450 693 B1**
(45) Date of publication and mention of the grant of the patent: **21.09.2005**
(21) Application number: 02787709.1
(22) Date of filing: 15.11.2002
(51) Int. Cl.: A61B 17/04, A61B 17/42, A61F 2/00

(54) **SURGICAL AUXILIARY INSTRUMENT**
CHIRURGISCHES HILFSINSTRUMENT
INSTRUMENT CHIRURGICAL AUXILIAIRE

(30) Priority: 03.12.2001 DE 10159181
(43) Date of publication of application: 01.09.2004
(73) Proprietor: Ethicon GmbH, 22851 Norderstedt (DE)
(72) Inventor: SALLER, Uwe, 21075 Hamburg (DE); LANDGREBE, Susanne, 23867 Süllfeld (DE); LUCENTE, Vincent, Allentown, PA 18104 (US)
(74) Representative: UEXKÜLL & STOLBERG
(86) International application number: PCT/EP2002/012849
(87) International publication number: WO 2003/047435

(56) References cited:
- WO-A-97/13465
- FR-A- 820 187
- US-A- 3 776 240

## Description

The invention relates to a surgical auxiliary instrument which can be used in particular in so-called TVT procedures.

The TVT surgical technique is a procedure for treating female incontinence as described e.g. in WO 96/06567 and WO 97/13465. A surgical instrument is used in which a strong bent surgical needle, which is guided with the help of a removable grip, is secured to each end of an implant tape made of polypropylene. The two needles are guided on opposite sides of the patient's urethra via the vagina along the rear side of the pubic bone to the outside of the abdominal wall. The tape comes to rest in a curve below the urethra. The two ends of the tape are pulled through the abdominal wall and cut off. As a rule, they need not be sewn as the tape grows in in the tissue relatively quickly. In the region of the urethra, the tape acts as a support without touching the urethra directly. The procedure is facilitated if the tape is provided with two covers which increase the gliding properties in the tissue and are removed from the tape at the end of the procedure via the two exit points of the tape. With this method, urinary incontinence can be treated quickly, effectively and in a manner causing little stress.

A correct position of the tape below the urethra is important. The tape must sit neither too tautly nor too slack. If the covers mentioned are removed from the tape towards the end of the procedure, there is a risk that the tape ends are taken along for a little with them and thus the tape below the urethra becomes too short and too taut. As a rule, the surgeon prevents this by inserting closed scissors between urethra and tape, with which he holds back the tape. In this manner he can also correct the position of the tape with the scissors in other phases of the procedure.

However, scissors are not a particularly suitable instrument for the named purpose. Handling is awkward and can even lead to injuries.

FR-A-820 187 discloses a surgical auxiliary instrument with a grip section in the proximal region, an adjoining intermediate section and an application section, in the distal region, set up for laying against an implant tape.

The object of the invention is to provide a possibility to facilitate the course of a procedure in which a tape is inserted, in particular a TVT procedure.

This object is achieved by a surgical auxiliary instrument with the features of claim 1. Advantageous designs of the invention result from the dependent claims.

The surgical auxiliary instrument according to the invention has a gripping section in the proximal zone, an adjoining intermediate section and an application section in the distal region set up for laying against an implant tape. If it is used during a TVT procedure, the application section is inserted between the patient's urethra and the pulled-in implant tape. Through the exertion of force, directed away from the urethra, on the auxiliary instrument, the position of the implant tape can be set, corrected or retained.

As the application section is set up for laying against the implant tape, the auxiliary instrument is much more suitable than scissors for checking the position of the tape below the urethra and ensuring a correct position. Thus the tape can be held with the auxiliary instrument when the covers are removed from the tape ends. Furthermore, it is possible to set and correct the position of the tape below the urethra and thus to prevent the tape tension from becoming too great and the tape from sitting too tautly. In principle, the auxiliary instrument according to the invention is used in similar manner to the aforementioned scissors, but because of its design, it is much more suitable for the purpose for which it is intended. In particular, injuries can be avoided by atraumatic shaping.

The application section preferably runs cranked relative to the grip section. This means that the intermediate section extends essentially at an obtuse angle relative to the grip section and the application section at a similar angle to the intermediate section, so that the grip section and the application section run largely parallel, but are offset relative to each other. This design is particularly suitable for use in a TVT procedure, as it makes possible a comfortable handling of the auxiliary instrument.

In the auxiliary instrument according to the invention, the application section contains a depression, the length of which, measured in longitudinal direction of the auxiliary instrument, is at least as great as the width of the implant tape to be used. This depression is open at both longitudinal sides of the auxiliary instrument. When the application section is inserted between the patient's urethra and the implant tape, the implant tape comes to rest in the depression and runs transversely with respect to the auxiliary instrument. The depression prevents the auxiliary instrument from slipping from the implant tape and thus increases reliability. When the application section runs cranked relative to the grip section, the depression is preferably accessible from the side on which the grip section is located. In this case, the advantages of the depression for a secure guiding of the implant tape and the cranked basic shape of the auxiliary instrument for a favourable position of the grip section come into play.

There are many possibilities for the design of the surgical auxiliary instrument according to the invention. The application section is preferably designed flat. The intermediate section can have a reinforcing profile which can also continue into the grip section. The auxiliary instrument is preferably manufactured from plastic as an injection moulding. In another version, it is bent into shape from a sheet metal part.

In the following, the invention is described in more detail using embodiments. The drawings show in
- Figure 1: a perspective view of a first version of the surgical auxiliary instrument according to the invention,
- Figure 2: a side view of the version from Figure 1, the cross-sectional shape of the auxiliary instrument at each of the points indicated by the dash-dot lines being given in the lower region of Figure 2,
- Figure 3: a perspective view of a second version of the surgical auxiliary instrument according to the invention and
- Figure 4: a perspective view of the version from Figure 3 as seen from a different angle.

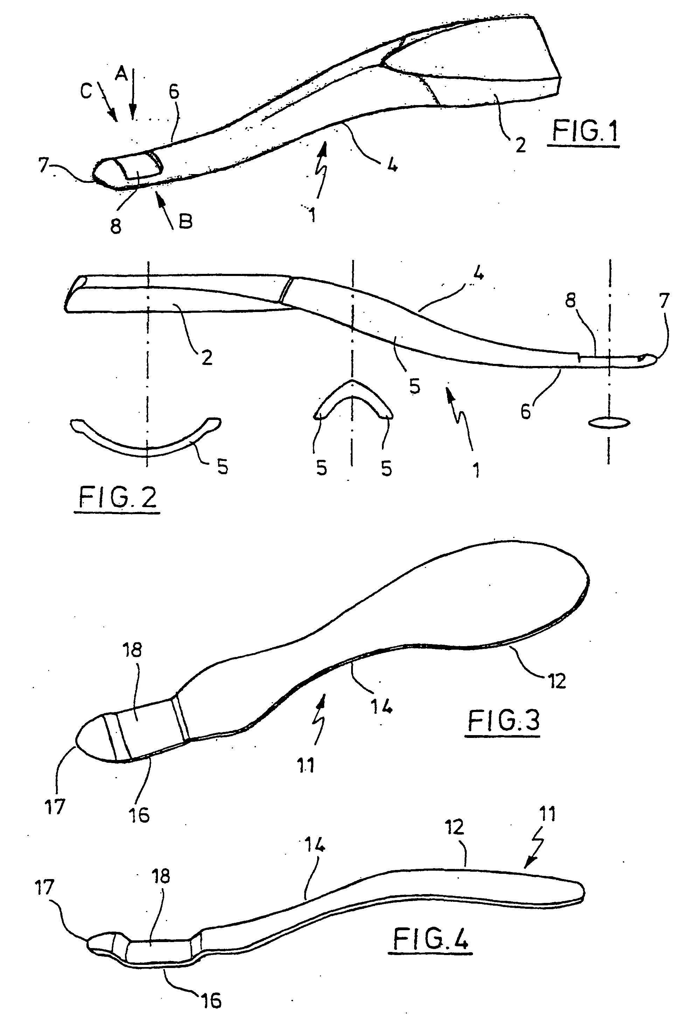

A first version of a surgical auxiliary instrument 1 is shown in Figures 1 and 2. The auxiliary instrument 1 contains in its proximal region a grip section 2 and adjoining it an intermediate section 4, which is provided with a profile 5 serving to reinforce it, which continues into the grip section 2. The distal region of the auxiliary instrument 1 is formed as an application section 6 and set up for laying against an implant tape. The distal end 7 of the auxiliary instrument 1 is rounded. Overall, the shape is atraumatic.

As can be seen in particular in Figure 2, the application section 6 runs cranked relative to the grip section 2. The application section 6 and the grip section 2 are therefore aligned largely parallel to each other but, because the intermediate section 4 extends at a slope, are offset relative to each other.

A depression 8 is formed in the application section 6. The length of the depression 8, measured in longitudinal direction of the auxiliary instrument 1, is somewhat greater than the width of an implant tape, the position of which can be set or corrected with the auxiliary instrument 1. The depression 8 starts from the side on which the grip section 2 is located, i.e. the top according to the representations in Figures 1 and 2. The depression 8 is therefore accessible from this side, i.e. in the direction of the arrow A. The depression 8 is open on the two longitudinal sides of the auxiliary instrument 1; it is therefore also accessible in the directions identified by arrows B and C. This design of the depression 8 makes it possible to accomodate the implant tape in the depression 8 when it runs transversely with respect to the auxiliary instrument 1, so that it rests securely against the application section 6 and cannot slip off.

The auxiliary instrument 1 is manufactured from plastic as an injection moulding and can be in one piece or several pieces. In the embodiment, it is in two pieces, the grip section 2 being inserted into the intermediate section 4.

Figures 3 and 4 show two perspective views of a second version of the auxiliary instrument, here numbered 11, as seen from different angles.

The auxiliary instrument 11 contains a grip section 12, an intermediate section 14 and an application section 16 with a distal end 17 and a depression 18. In their function, these parts correspond to the parts explained using Figures 1 and 2. The application section 16 again runs cranked relative to the grip section 12. In contrast to the auxiliary instrument 1, the auxiliary instrument 11 is shaped from a piece of sheet metal.

The surgical auxiliary instrument is suitable in particular for use in a TVT procedure, as explained in the following using the auxiliary instrument 1. The surgical technique itself is summarized at the beginning and described in more detail in the documents cited there. When the implant tape has been pulled in and lies below the patient's urethra, the surgeon must check and if necessary correct the position of the tape. To this end, he pushes the auxiliary instrument 1 between the urethra and the tape so that the tape comes to rest in the depression 8 and runs transversely with respect to the auxiliary instrument 1. Through pressure onto the grip section 2, he can increase the distance between the urethra and the tape, in order to thus optimally set or correct the position of the tape. If, towards the end of the procedure, the covers mentioned at the beginning are pulled off from the ends of the implant tape, the surgeon can, with the auxiliary instrument 1, prevent the tape from being taken along and consequently lying too tightly against the urethra.

The cranked shape of the auxiliary instrument 1 is geared to use in a TVT procedure. It enables the application section 6 to be located in the desired operating area whilst the grip section 2 lies outside and can be gripped without difficulty.

## Claims

1. Surgical auxiliary instrument with a grip section (2; 12) in the proximal region, an adjoining intermediate section (4; 14) and an application section (6; 16), in the distal region, set up for laying against an implant tape, **characterized in that** the application section (6; 16) has a depression (8; 18), the length of which, measured in longitudinal direction of the auxiliary instrument (1; 11), is at least as great as the width of the implant tape to be used, and which is open on both longitudinal sides of the auxiliary instrument (1; 11).

2. Surgical auxiliary instrument according to claim 1, **characterized in that** the application section (6; 16) runs cranked relative to the grip section (2; 12).

3. Surgical auxiliary instrument according to claim 2, **characterized in that** the depression (8; 18) is accessible from the side on which the grip section (2; 12) is located.

4. Surgical auxiliary instrument according to one of claims 1 to 3, **characterized in that** the application section (6; 16) is designed flat.

5. Surgical auxiliary instrument according to one of claims 1 to 4, **characterized in that** the intermediate section (4) has a reinforcement profile (5).

6. Surgical auxiliary instrument according to one of claims 1 to 5, **characterized in that** it is designed as an injection moulding (1).

## Patentansprüche

1. Chirurgisches Hilfsinstrument, mit einem Griffabschnitt (2; 12) im proximalen Bereich, einem daran anschließenden Zwischenabschnitt (4; 14) und einem zum Anlegen an einem Implantatband eingerichteten Anlageabschnitt (6; 16) im distalen Bereich, **dadurch gekennzeichnet, dass** der Anlageabschnitt (6; 16) eine Vertiefung (8; 18) aufweist, deren Länge, in Längsrichtung des Hilfsinstruments (1; 11) gemessen, mindestens so groß ist wie die Breite des anzuwendenden Implantatbands und die zu beiden Längsseiten des Hilfsinstruments (1; 11) offensteht.

2. Chirurgisches Hilfsinstrument nach Anspruch 1, **dadurch gekennzeichnet, dass** der Anlageabschnitt (6; 16) in bezug auf den Griffabschnitt (2; 12) gekröpft verläuft.

3. Chirurgisches Hilfsinstrument nach Anspruch 2, **dadurch gekennzeichnet, dass** die Vertiefung (8; 18) von der Seite aus zugänglich ist, auf der sich der Griffabschnitt (2; 12) befindet.

4. Chirurgisches Hilfsinstrument nach einem der Ansprüche 1 bis 3, **dadurch gekennzeichnet, dass** der Anlageabschnitt (6; 16) flach ausgestaltet ist.

5. Chirurgisches Hilfsinstrument nach einem der Ansprüche 1 bis 4, **dadurch gekennzeichnet, dass** der Zwischenabschnitt (4) eine Verstärkungsprofilierung (5) aufweist.

6. Chirurgisches Hilfsinstrument nach einem der Ansprüche 1 bis 5, **dadurch gekennzeichnet, dass** es als Spritzteil (1) ausgestaltet ist.

## Revendications

1. Instrument chirurgical auxiliaire avec une section de prise (2 ; 12) dans la région proximale, une section intermédiaire attenante (4 ; 14) et une section d'application (6 ; 16) dans la région distale installée pour se trouver contre une bande d'implant, **caractérisé en ce que** la section d'application (6 ; 16) possède une dépression (8 ; 18) dont la longueur, mesurée dans la direction longitudinale de l'instrument auxiliaire (1 ; 11), est au moins aussi grande que la largeur de la bande d'implant à utiliser, et qui est ouverte des deux cotés longitudinaux de l'instrument auxiliaire (1 ; 11).

2. Instrument chirurgical auxiliaire selon la revendication 1, **caractérisé en ce que** la section d'application (6 ; 16) s'étend coudée par rapport à la section de prise (2 ; 12).

3. Instrument chirurgical auxiliaire selon la revendication 2, **caractérisé en ce que** la dépression (8 ; 18) est accessible à partir du côté sur lequel la section de prise (2 ; 12) est située.

4. Instrument chirurgical auxiliaire selon l'une des revendications 1 à 3, **caractérisé en ce que** la section d'application (6 ; 16) est conçue plate.

5. Instrument chirurgical auxiliaire selon l'une des revendications 1 à 4, **caractérisé en ce que** la section intermédiaire (4) possède un profil de renforcement (5).

6. Instrument chirurgical auxiliaire selon l'une des revendications 1 à 5, **caractérisé en ce qu'**il est conçu par moulage par injection.
